(19) Europäisches Patentamt
European Patent Office
Office européen des brevets

(11) Veröffentlichungsnummer: **0 387 974 A2**

(12) # EUROPÄISCHE PATENTANMELDUNG

(21) Anmeldenummer: 90250062.8

(22) Anmeldetag: 07.03.90

(51) Int. Cl.[5]: C07C 69/635, C07C 67/08, A01N 37/02, C07C 67/14, C07C 67/343, C07C 69/65, C07C 69/734, C07C 233/15, C07C 53/23, C07C 53/50

(30) Priorität: 15.03.89 DE 3908901

(43) Veröffentlichungstag der Anmeldung: 19.09.90 Patentblatt 90/38

(84) Benannte Vertragsstaaten: AT BE CH DE DK ES FR GB GR IT LI LU NL SE

(71) Anmelder: SCHERING AKTIENGESELLSCHAFT
Müllerstrasse 170/178
D-1000 Berlin 65(DE)

(72) Erfinder: Hömberger, Günter, Dr.
Waldmannstrasse 21
D-1000 Berlin 46(DE)
Erfinder: Köhn, Arnim, Dr.
Alt-Wittenau 63c
D-1000 Berlin 26(DE)
Erfinder: Wegner, Peter, Dr.
Münchener Str. 3
D-1000 Berlin 28(DE)
Erfinder: Keyserlingk, Harald v., Dr.
Markgrafenstrasse 38
D-1000 Berlin 28(DE)

(54) **2-Halogencyclopropylethan-derivate, Verfahren zu ihrer Herstellung und ihre Verwendung als Schädlingsbekämpfungsmittel.**

(57) Es werden neue 2-Halogencyclopropylethanderivate der allgemeinen Formel I

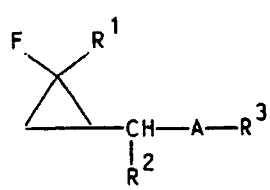

(I),

und neue Zwischenprodukte der allgemeinen Formel II

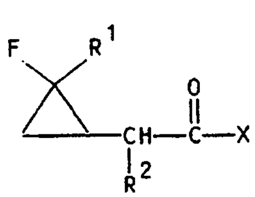

(II),

in denen $R^1$, $R^2$, $R^3$, A und X die in der Beschreibung genannten Bedeutungen haben, sowie Verfahren zur Herstellung der Verbindungen der allgemeinen Formel I beschrieben.
Die erfindungsgemäßen Verbindungen können als Schädlingsbekämpfungsmittel, insbesondere gegen In-

EP 0 387 974 A2

sekten und Milben, verwendet werden.

# 2-Halogencyclopropylethanderivate, Verfahren zu ihrer Herstellung und ihre Verwendung als Schädlingsbekämpfungsmittel

Die Erfindung betrifft neue 2-Halogencyclopropylethanderivate, ihre Herstellung und ihre Verwendung als Schädlingsbekämpfungsmittel, insbesondere gegen Insekten und Milben.

Es ist bereits bekannt, daß Cyclopropanverbindungen akarizide und insektizide Eigenschaften besitzen (EP 116 889).

Der Nachteil der bekannten Verbindungen ist jedoch die nicht ausreichende insektizide und akarizide Wirksamkeit.

Die Aufgabe der vorliegenden Erfindung ist es, Verbindungen zur Verfügung zu stellen, welche Insekten und Milben besser als die für diesen Zweck bekannten Verbindungen bekämpfen.

Es wurde nun gefunden, daß 2-Halogencyclopropylethanderivate der allgemeinen Formel I

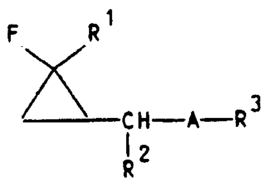

(I),

in der

$R^1$ Wasserstoff oder Chlor,

$R^2$ Wasserstoff, $C_1$-$C_6$-Alkyl, Aryl oder Aryl-$C_1$-$C_2$-alkyl,

A die Gruppe $-CH_2-B-\underset{\underset{D}{\|}}{C}-$

oder $-\underset{\underset{D}{\|}}{C}-E-$

(hierbei sind

$B-\overset{\overset{D}{\|}}{C}-$ oder E- mit $R^3$ verbunden, wobei B und D Sauerstoff oder Schwefel bedeuten),

E Sauerstoff, Schwefel oder den Rest $NR^4$ bedeutet und

$R^3$ und R unabhängig voneinander für Wasserstoff, ein Akalimetallatom oder ein entsprechendes Äquivalent eines zweiwertigen Metalls, $C_1$-$C_{20}$-Alkyl, $C_2$-$C_{20}$-Alkenyl $C_2$-$C_{20}$-Alkinyl, Halogen-$C_1$-$C_{10}$-alkyl, $C_3$-$C_6$-Cycloalkyl, $C_1$-$C_3$-Alkyl-$C_3$-$C_6$-cycloalkyl, $C_3$-$C_6$-Cycloalkyl-$C_1$-$C_6$-alkyl, Halogen-$C_3$-$C_6$-cycloalkyl-$C_1$-$C_6$-alkyl, Bicycloalkyl, Chlorfluorcyclopropylmethylcarbonyloxy-$C_1$-$C_{10}$-alkyl, Chlorfluorcyclopropylcarbonyloxy-$C_1$-$C_3$-alkoxy-$C_1$-$C_3$-alkyl, Aryl-$C_1$-$C_6$-alkyl, Aryl-$C_2$-$C_6$-alkenyl, Halogenaryl-$C_1$-$C_6$-alkyl, $C_1$-$C_4$-Alkylaryl-$C_1$-$C_4$-alkyl, Halogenaryl-$C_2$-$C_6$-alkenyl, Halogen-$C_1$-$C_4$-alkylaryl-$C_1$-$C_6$-alkyl, $C_1$-$C_3$-Alkoxyaryl-$C_1$-$C_6$-alkyl, Aryloxybenzyl, $\alpha$-$C_1$-$C_3$-Alkylphenoxybenzyl, Halogenphenyl(cyclopropyl)-$C_1$-$C_3$-alkyl, Halogenphenoxy-$C_1$-$C_6$-alkyl, Naphthyl-$C_1$-$C_6$-alkyl, freies Aryl oder durch $C_1$-$C_{20}$-Alkyl, Halogen-$C_1$-$C_6$-alkyl, $C_1$-$C_{16}$-Alkoxy, Halogen-$C_1$-$C_6$-alkoxy, Phenyl-$C_1$-$C_6$-alkyl, Phenyl-$C_1$-$C_6$-alkoxy, $C_3$-$C_{10}$-Cycloalkoxy, Halogen-$C_3$-$C_{10}$-cycloaloxy, $C_3$-$C_6$-Cycloalkylalkyloxy, Halogen-$C_3$-$C_6$-cycloalkyalkyloxy, $C_2$-$C_6$-Alkenyloxy, Halogen-$C_2$-$C_6$-alkenyloxy, $C_2$-$C_6$-Alkinyloxy, Halogen-$C_2$-$C_6$-alkinyloxy, Alkylsulfonyloxy, Alkylphenylsulfonyloxy, Halogenalkylsulfonyloxy, Phenyl, Halogen, Amino, Cyano, Hydroxy, Nitro, Aryloxy, Heteroaryloxy, Halogenaryloxy, Arylamino, Halogenarylamino, $C_1$-$C_6$-Alkoxycarbonyl, $C_1$-$C_6$-Alkoxycarbonylmethyl, Halogen-$C_1$-$C_6$-alkoxycarbonyl, $C_1$-$C_2$-Alkyldioxy, $C_1$-$C_6$-Alkylthio, Halogen-$C_3$-$C_6$-cycloalkylalkylamino, Halogen-$C_3$-$C_6$-cycloalkylalkylcarbonyloxy, $C_1$-$C_6$-Alkylamino, oder Di-$C_1$-$C_6$-alkylamino ein- oder mehrfach substituiertes Aryl, freies Heteroaryl, durch Halogen, $C_1$-$C_3$-Alkyl oder für Halogen-$C_1$-$C_3$-alkyl substituiertes Heteroalkyl stehen,

eine im Vergleich zu den bekannten Verbindungen verbesserte insektizide und akarizide Wirksamkeit zeigen.

Die Bezeichnung "Alkyl" steht für eine geradlinige oder verzweigte Kette von Kohlenstoffatomen.

Die Bezeichnung "Alkenyl" steht für eine geradlinige oder verzweigte Kette von Kohlenstoffatomen, die durch Doppelbindungen ein- oder mehrfach unterbrochen ist.

Die Bezeichnung "Alkinyl" steht für eine geradlinige oder verzweigte Kette von Kohlenstoffatomen, die durch Dreifachbindungen ein- oder mehrfach unterbrochen ist.

Die Bezeichnung "Aryl" steht für einen ein- bis dreikernigen aromatischen Rest, wie z.B. Phenyl, Naphthyl oder Phenanthryl.

Die Bezeichnung "Heteroaryl" steht für einen 5- oder 6-gliedrigen Ring, der ein oder mehrere Stickstoff-, Sauerstoff- und/oder Schwefelatome enthält, gesättigt, teilgesättigt oder ungesättigt sein kann und gegebenenfalls benzoanelliert ist, wie z.B. Pyridin, Thiazol oder Chromen.

$R^3$ und $R^4$ können gegebenenfalls gemeinsam mit dem N-Atom gesättigte oder ungesättigte heterocyclische Ringe darstellen, wie z.B. Morpholino, Piperidino, Pyrrolo, Imidazolo oder Triazolo.

Die erfindungsgemäßen Verbindungen der allgemeinen Formel I liegen als Gemische der optisch aktiven Isomeren vor. Die Erfindung betrifft nicht nur die Isomerengemische, sondern auch jedes einzelne Isomer der erfindungsgemäßen Verbindungen.

Die Erfindung betrifft ferner Verbindungen der allgemeinen Formel II

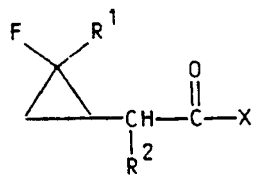

(II),

in der

$R^1$ und $R^2$ die in der Formel I angegebene Bedeutung haben und

X für Hydroxy, Chlor oder Brom steht, als Zwischenprodukte zur Herstellung von Verbindungen der Formel I.

Die neuen Zwischenprodukte eigenen sich insbesondere vorteilhaft zur Herstellung der erfindungsgemäßen Verbindungen der Formel I, bei denen

A für die Gruppe $-\underset{\underset{O}{\parallel}}{C}-E$

steht.

Die erfindungsgemäßen 2-Halogencyclopropylethanderivate der Formel I, bei denen

A für die Gruppe

$-\overset{\overset{O}{\parallel}}{C}-E-$ steht, lassen sich herstellen, indem man entweder

A) Ein Säurehalogenid der allgemeinen Formel II

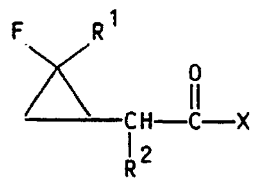

(II),

in der

$R^1$ und $R^2$ die in Formel I angegebene Bedeutung haben und

X für Chlor oder Brom steht,

mit einem Alkohol oder Amin der allgemeinen Formel III

$$H - E - R^3 \quad (III),$$

in der

E und $R^3$ die in Formel I angegebene Bedeutung haben, gegebenenfalls in einem Lösungsmittel in Gegenwart eines Säureacceptors umsetzt, oder

B) eine freie Säure der allgemeinen Formel IV

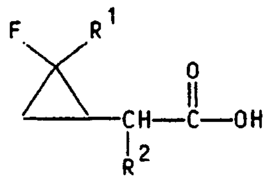 (IV),

in der
$R^1$ und $R^2$ die in Formel I angegebene Bedeutung haben, mit einem Alkohol oder Amin der Formel III, gegebenenfalls unter Verwendung eines Lösungsmittels in Gegenwart eines Katalysators, umsetzt, oder

C) eine Säure der allgemeinen Formel V

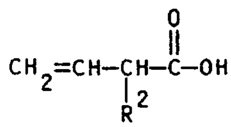 (V),

in der
$R^2$ die in Formel I angegebene Bedeutung hat, mit einem Alkohol oder Amin der Formel III, gegebenenfalls in einem Lösungsmittel in Gegenwart eines Katalysators oder wasserentziehender Mittel, zu einer Zwischenverbindung der Formel VI

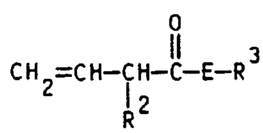 (VI),

in der
E, $R^2$ und $R^3$ die in Formel I angegebene Bedeutung haben, umsetzt und diese in Gegenwart eines inerten Lösungsmittels mit Chlorfluorcarben reagieren läßt, oder falls
A die Gruppe $-CH_2-B-\underset{\underset{D}{\|}}{C}-$
bedeutet,

D) einen Alkohol der allgemeinen Formel VII

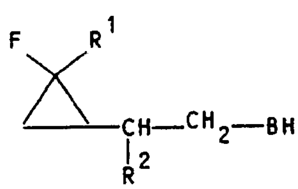 (VII),

in der
$R^1$, $R^2$ und B die in Formel I angegebene Bedeutung haben, mit einer Säure der allgemeinen Formel VIII
$R^3-\underset{\underset{D}{\|}}{C}-BH$ (VIII),
in der
B, D und $R^3$ die in Formel I angegebene Bedeutung haben, gegebenenfalls unter Verwendung eines Lösungsmittels sowie eines Katalysators, umsetzt, oder

E) einen Alkohol der allgemeinen Formel VII mit einem Säurehalogenid der allgemeinen Formel IX
$R^3-\underset{\underset{O}{\|}}{C}-X$ (IX),
in der
X für Chlor oder Brom steht und

$R^3$ die in Formel I angegebene Bedeutung hat, gegebenenfalls unter Verwendung eines Lösungsmittels sowie eines Säureacceptors, umsetzt, oder

F) einen Alkohol der allgemeinen Formel X

$$CH_2 = CH - \underset{R^2}{\underset{|}{C}}H - CH_2 - BH \qquad (X),$$

in der

B und $R^2$ die in Formel I angegebene Bedeutung haben, mit einer Säure der allgemeinen Formel VIII, gegebenenfalls unter Verwendung eines Lösungsmittels sowie eines Katalysators, zu einer Zwischenverbindung der Formel XI

$$CH_2 = CH - \underset{R^2}{\underset{|}{C}}H - CH_2 - B - \overset{D}{\overset{\|}{C}} - R^3 \qquad (XI),$$

in der

B, D, $R^2$ und $R^3$ die in Formel I angegebene Bedeutung haben, umsetzt und diese in Gegenwart eines inerten Lösungsmittels mit Chlorfluorcarben reagieren läßt, oder

G) einen Alkohol der allgemeinen Formel X mit einem Säurehalogenid der allgemeinen Formel IX, gegebenenfalls unter Verwendung eines Lösungsmittels sowie eines Säureacceptors, zu einer Zwischenverbindung der Formel XI umsetzt und diese in Gegenwart eines inerten Lösungsmittels mit Chlorfluorcarben reagieren läßt.

Als Säureacceptor für die Durchführung der Reaktionsvarianten A), E) und G) eignen sich die üblichen basischen Mittel, insbesondere aliphatische, aromatische und heterocyclische Amine, wie z.B. Triethylamin, Dimethylanilin, Dimethylbenzylamin, Pyridin und Dimethylaminopyridin oder anorganische Basen wie Oxide, Carbonate, Hydrogencarbonate und Alkoholate von Alkali- und Erdalkalimetallen wie Kaliumhydroxid, Natriumhydroxid, Natrium- und Kaliumcarbonat.

Als Lösungsmittel eignen sich die vorgenannten Säureacceptoren selbst oder inerte Lösungsmittel oder Gemische derselben untereinander.

Genannt seien beispielsweise aliphatische, alicyclische und aromatische Kohlenwasserstoffe, die gegebenenfalls chloriert sein können wie Hexan, Cyclohexan, Petrolether, Benzol, Toluol, Xylol, Methylenchlorid, Chloroform, Kohlenstofftetrachlorid, Ethylenchlorid, Trichlorethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Benzonitril; Ester wie Ethylacetat und Amylacetat; Säureamide wie Dimethylformamid und Dimethylacetamid sowie Sulfone und Sulfoxide wie Dimethylsulfoxid und Sulfolan.

Die Umsetzung kann innerhalb eines weiten Temperaturbereiches durchgeführt werden. Im allgemeinen wird sie bei einer Temperatur zwischen -20 °C und 200 °C, durchgeführt.

Die Umsetzung wird unter dem Druck der Umgebung durchgeführt, wenngleich sie auch bei erhöhtem Druck oder vermindertem Druck durchgeführt werden könnte.

Als Katalysatoren für die Durchführung der Reaktionsvarianten B), D) und F) eignen sich starke Säuren wie Schwefelsäure, Halogenwasserstoff, Sulfonsäuren und saure Ionenaustauscher. Es ist vorteilhaft, dem Reaktionsgemisch das Wasser oder den Ester der Formel I zu entziehen, z.B. durch azeotrope Destillation oder durch Bindung des Wassers an Schwefel- oder Halogenwasserstoffsäure.

Die Reaktionsvariante B) kann unter den gleichen Reaktionsbedingungen in Bezug auf Temperatur und Druck und in den gleichen Lösungsmitteln oder Gemischen derselben durchgeführt werden wie sie für die Reaktionsvariante A) genannt wurden.

Für die Durchführung der Reaktionsvariante C) eignen sich zur Herstellung der Zwischenverbindung VI die gleichen sauren Katalysatoren und inerten Lösungsmittel wie die, die für Reaktionsvariante B) genannt wurden. Besonders geeignet für die Veresterung ist die Bindung des Wassers durch die Kombination von Triphenylphosphin und Azodicarbonsäureester (Synthesis 1981, 1). Geeignet sind aber auch die klassischen wasserentziehenden Mittel wie konzentrierte Schwefelsäure, wasserfreie Salze von anorganischen Säuren wie Magnesiumsulfat oder Calciumchlorid, Carbodiimide wie das Dicyclohexylcarbodiimid oder auch Zeolithe.

Die Erzeugung von Chlorfluorcarben erfolgt nach an sich bekannten Methoden (z.B. Wakselman et al. Synthesis 1985, 754). Bevorzugt wird jedoch die Darstellung von Chlorfluorcarben aus Salzen der Dichlorfluoressigsäure in hochsiedenden Lösungsmitteln wie Diglyme, Triglyme und Tetraglyme.

Als Chlorfluorcarbendonatoren eignen sich beispielsweise Halogenfluorkohlenwaserstoffe wie Dichlorfluormethan und Alkalimetalldichlorfluoracetate wie Natriumdichlorfluoracetat.

Die Herstellung der optischen Isomeren der erfindungsgemäßen Verbindungen kann nach an sich bekannten Verfahren durchgeführt werden, beispielsweise durch Umsetzen von Verbindungen der allgemeinen Formel II mit einem chiralen Hilfsreagenz, wie z.B. einem optisch aktiven Amin oder einem optisch aktiven Alkohol und anschließender Trennung der so erhaltenen Diastereomeren mit Hilfe physi kalischer Methoden (Tetrahedron 33, 2725 (1977) ) , wie z. B. Kristallisation, Destillation oder Fest-Flüssig-Chromatographie. Durch eine anschließende hydrolytische Spaltung, die entweder säure- oder basenkatalytisch geführt werden kann, erhält man die optischen Isomeren der freien Säuren der allgemeinen Formel IV, die nach Verfahrensvariante B) zu den erfindungsgemäßen Verbindungen umgesetzt werden können.

Weiterhin können die bei der Synthese entstehenden Gemische optischer Isomere der allgemeinen Formel I durch Chromatographie an chiralen stationären Phasen, wie z.B. an Cyclodextrinen, Stärke oder an Polymere gebundene optisch aktive Aminosäurederivate, in die Enantiomere getrennt werden (Angew. Chem. 92, 14 (1980)).

Die nach oben genannten Verfahren herstellbaren erfindungsgemäßen Verbindungen können nach den üblichen Verfahren aus dem Reaktionsgemisch isoliert werden, beispielsweise durch Abdestillieren des eingsetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und geruchlose Flüssigkeiten sowie Kristalle dar, die schwerlöslich in Wasser, bedingt löslich in aliphatischen Kohlenwasserstoffen wie Petrolether, Hexan, Pentan und Cyclohexan, gut löslich in halogenierten Kohlenwasserstoffen wie Chloroform, Methylenchlorid und Tetrachlorkohlenstoff, aromatischen Kohlenwasserstoffen wie Benzol, Toluol und Xylol, Ethern wie Diethylether, Tetrahydrofuran und Dioxan, Carbonsäurenitrilen wie Acetonitril, Alkoholen wie Methanol und Ethanol, Carbonsäureamiden wie Dimethylformamid und Sulfoxiden wie Dimetehylsulfoxid, sind.

Die als Ausgangsmaterial zu verwendenden Säurehalogenide der Formel IX, die Säure der Formel VIII, der Alkohol und das Amin der Formel III sind bekannt oder können nach an sich bekannten Methoden hergestellt werden.

Die erfindungsgemäßen Verbindungen zeichnen sich durch gute insektizide und akarizide Wirkung aus und stellen damit eine wertvolle Bereicherung der Technik dar. Aufgrund ihrer Wirkung gegen eine weite Spanne saugender Arthropoden unterschiedlichster Verwandschaftsgruppen können die erfindungsgemäßen Verbindungen nicht nur gegen Schädlinge an Kulturpflanzen eingesetzt werden, sondern auch zur Bekämpfung von Parasiten des Menschen und der Nutztiere. Besondere Bedeutung hat die Wirkung der erfindungsgemäßen Verbindungen gegen Parasiten, die hinsichtlich anderer Mittel Resistenz entwickelt haben.

Zu den Insekten und Milben, einschließlich tierischer Ektoparasiten, die erfindungsgemäß bekämpft werden können, gehören beispielsweise die Lepidopteren wie Plutella xylostella, Spodoptera littoralis, Heliothis armigera und Pieris brassicae; die Dipteren wie Musca domestica, Ceratitis capitata, Erioischia brassicae, Lucilia sericata und Aedes aegypti; die Homopteren einschließlich Blattlausen wie Megoura viciae und Nilaparvata lugens; die Coleopteren wie Phaedon cochleariae, Anthonomus grandis und Cornrootworm (Diabrotica spp., beispielsweise Diabrotica undecimpunctata) die Othopteren wie Blattella germanica; die Zecken wie Boophilus microplus und die Lause wie Damalinia bovis und Linognathus vituli sowie die Spinnmilben wie Tetranychus urticae und Panonychus ulmi.

Die Anwendung der erfindungsgemaßen Verbindungen kann in Konzentrationen von 0,0005 bis 5,0 %, vorzugsweise von 0,001 bis 0,1 % erfolgen, worunter das Gewicht in Gramm Wirkstoff in 100 ml Zubereitung zu verstehen ist.

Die erfindungsgemäßen Verbindungen können entweder allein, in Mischung miteinander oder mit anderen insektiziden Wirkstoffen angewendet werden. Gegebenenfalls können andere Pflanzenschutz- oder Schädlingsbekämpfungsmittel wie zum Beispiel Insektizide, Akarizide oder Fungizide, je nach dem gewünschten Zweck zugesetzt werden.

Eine Förderung der Wirkungsintensität und der Wirkungsgeschwindigkeit kann zum Beispiel durch wirkungssteigernde Zusätze wie organische Lösungsmittel. Netzmittel und Öle erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der

Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßig werden die erfindungsgemäßen Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Tonsil, Silicagel, Talkum, Kaolin, Attapulgit, Kalkstein und pflanzliche Produkte, zum Beispiel Mehle.

An oberflächenaktiven Stoffen sind zum Beispiel zu nennen Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozente Wirkstoff, etwa 90 bis 10 Gewichtsprozente flüssige oder feste Trägerstoffe sowie gegebenenfalls bis zu 20 Gewichtsprozente oberflächenaktive Stoffe.

Die Ausbringung der Mittel kann in üblicher Weise erfolgen, zum Beispiel mit Wasser als Träger in Spritzbrühmengen von etwa 100 bis 3.000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Zur Herstellung der Zubereitungen werden zum Beispiel die folgenden Bestandteile eingesetzt:

A SPRITZPULVER

20 Gew.-% Wirkstoff
35 Gew.-% Bleicherde
8 Gew.-% Calciumsalz der Ligninsulfonsäure
2 Gew.-% Natriumsalz des N-Methyl-N-oleyl-taurins
35 Gew.-% Kieselsäure

B PASTE

45 Gew.-% Wirkstoff
5 Gew.-% Natriumaluminiumsilikat
15 Gew.-% Cetylpolyglycolether mit
8 Mol Ethylenoxid
2 Gew.-% Spindelöl
10 Gew.-% Polyethylenglykol
23 Teile Wasser

C EMULSIONSKONZENTRAT

20 Gew.-% Wirkstoff
75 Gew.-% Isophoron
5 Gew.-% einer Mischung auf Basis des Natriumsalzes des N-Methyl-N-oleyl-taurins und des Calciumsalzes der Ligninsulfonsäure. Gew.-% = Gewichtsprozent

Die folgenden Beispiele beschreiben die Herstellung der erfindungsgemäßen Verbindungen.

**Beispiel 1**

4-(2-Propyloxy)-benzoesäure-[2-(2-chlor-2-fluorcyclopropyl)-ethyl]-ester

1. Zu 25 g (0,14 mol) 4-(2-Propyloxy)-benzoesäure und 22,5 ml (0,14 mol) Azodicarbonsäurediethylester in 150 ml Tetrahydrofuran tropft man unter Kühlung eine Lösung von 35,9 g (0,14 mol) Triphenylphosphin und 15,5 ml (0,18 mol) 3-Buten-1-ol in 150 ml Tetrahydrofuran und rührt 5 Stunden bei Raumtemperatur nach. Nach Verdampfen des Lösungsmittels wird das Reaktionsprodukt säulenchromatographisch an Kieselgel, mit Hexan/Essigester 95:5 als Elutionsmittel gereinigt und eine Probe dünnschichtchromatographisch, mit Hexan/Essigester 8:2 als Elutionsmittel analysiert ($R_f$: 0,61).
Es werden 29 g (89 % der Theorie) 4-(2-Propyloxy)-benzoesäure-3-buten-1-ylester erhalten.

2. In eine heftig gerührte Mischung aus 21 g (0,09 mol) 4-(2-Propyloxy)-benzoesäure-3-buten-1-

ylester, 100 ml 50 %ige Natronlauge, 150 ml Dichlormethan und 1 g (4,4 mmol) Triethylbenzylammoniumchlorid werden bei 5 bis 10 °C 60 g (0,58 mol) Dichlorfluormethan (Freon 21) über einen Zeitraum von 1 bis 2 Stunden eingeleitet. Anschließend wird bei 5 °C 3 Stunden nachgerührt. Das Reaktionsgemisch wird auf 500 ml Wasser gegossen und zweimal mit je 100 ml Methylenchlorid extrahiert. Die vereinigten organischen Phasen werden mit Wasser gewaschen und über Magnesiumsulfat getrocknet. Das Rohprodukt wird nach Abdampfen des Lösungsmittels säulenchromatographisch an Kieselgel, mit Hexan/Essigester 95:5 als Elutionsmittel, gereinigt und eine Probe dünnschichtchromatographisch, mit Hexan/Essigester = 8:2 als Elutionsmittel analysiert ($R_f$ = 0,53).

Es werden 4,5 g (16 % der Theorie) 4-(2-Propyloxy)-benzoesäure-[-2-(2-chlor-2-fluorcyclopropyl)-ethyl]-ester erhalten.

$n_D^{20}$ : 1,5139

**Beispiel 2**

2-(2-Chlor-2-fluorcyclopropyl)-essigsäure-(2-naphthylmethyl)-ester

Eine Lösung von 3,61 g (0,016 mol) 3-Butensäure-(2-naphthylmethyl)-ester in 40 ml Diglyme wird auf 162 °C erhitzt. Bei dieser Temperatur wird eine Lösung von 8,1 g (0,048 mol) Natriumdichlorfluoracetat in 60 ml Diglyme über einen Zeitraum von 1 Stunde hinzugetropft. Danach läßt man abkühlen, destilliert das Lösungsmittel im Ölpumpenvakuum ab, nimmt den Rückstand in Essigester auf, wäscht zweimal mit Wasser, trocknet über Magnesiumsulfat und engt am Rotationsverdampfer ein. Das Rohprodukt wird säulenchromatographisch an Kieselgel, mit Hexan/Essigester 99:1 als Elutionsmittel, gereinigt und eine Probe wird dünnschichtchromatographisch, mit Hexan/Essigester 8:2 als Elutionsmittel, analysiert $R_f$ : 0,39).

Es werden 1,7 g (36 % der Theorie) 2-(2-Chlor-2-fluorcyclopropyl)-essigsäure(2-naphthylmethyl)-ester erhalten.

$n_D^{20}$ : 1,5669

In analoger Verfahrensweise lassen sich die folgenden Verbindungen herstellen.

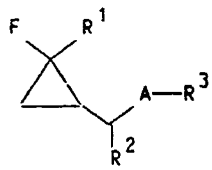

$R^1$ = Cl
$R^2$ = H

| Beispiel | Verfahrens-variante | A | $R^3$ | $n_D^{20}$ bzw. Fp. [°C] |
|---|---|---|---|---|
| 3 | C | 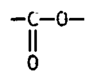 | 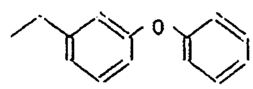 | 1,5491 |
| 4 | C | 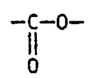 | n-$C_{16}H_{33}$ | 1,4509 |
| 5 | F | 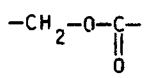 | 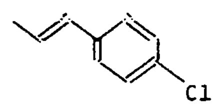 | 1,5576 |
| 6 | C | 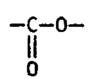 | 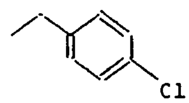 | 1,5164 |
| 7 | C | 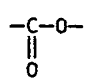 | 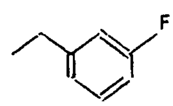 | 1,4911 |
| 8 | C | 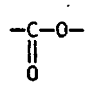 | 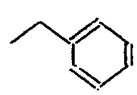 | 1,5031 |

| Beispiel | Verfahrens-variante | A | $R^3$ | $n_D^{20}$ bzw. Fp. [°C] |
|---|---|---|---|---|
| 9 | F | 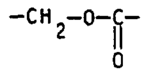 | 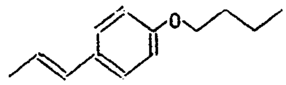 | 1,5385 |
| 10 | | 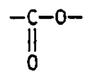 | H | ($R_f$ = 0,15 in Essig-ester) |
| 11 | A | 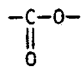 | 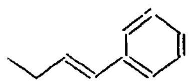 | 1,5338 |
| 12 | A | 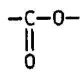 | 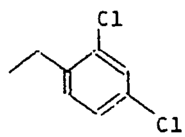 | 1,5303 |
| 13 | A | 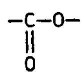 | 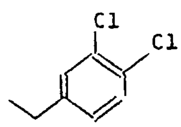 | 1,5313 |
| 14 | A | 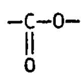 | 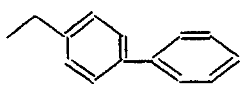 | 1,5695 |
| 15 | A | 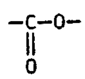 | 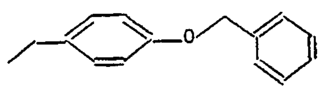 | 1,5497 |
| 16 | A | 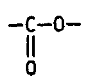 | 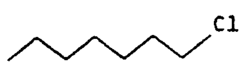 | 1,4584 |

| Beispiel | Verfahrens-variante | A | $R^3$ | $n_D^{20}$ bzw. Fp. [°C] |
|---|---|---|---|---|
| 17 | A | 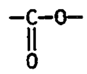 | 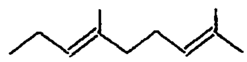 | 1,4717 |
| 18 | A | 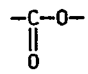 | 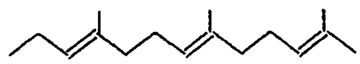 | 1,4809 |
| 19 | A | 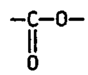 | 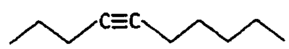 | 1,4585 |
| 20 | A | 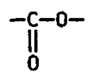 | 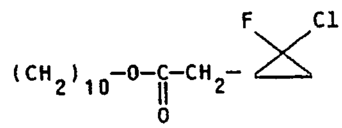 | 1,4579 |
| 21 | A | 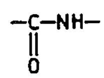 | 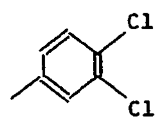 | 101,4 |
| 22 | A | 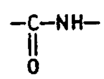 | 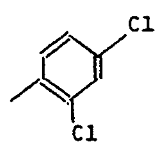 | 79,6 |
| 23 | F | 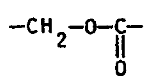 | 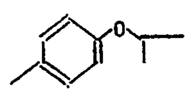 *) | 1,5118 |
| 24 | F | 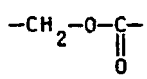 | 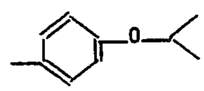 **) | 1,5150 |

*) Z-Form

**) E-Form

Die nachfolgenden Beispiele zeigen die Wirksamkeit der erfindungsgemäßen Verbindungen.

## Anwendungsbeispiel A

Wirkung der prophylaktischen Futterbehandlung gegen die Schwarze Bohnenlaus (Aphis fabae Scop.)

Aus entwickelten Primärblättern der Buschbohne (Phaseolus vulgaris nanus Aschers.) werden runde Blattscheiben mit einem Durchmesser von 24 mm gestanzt und unbehandelt bzw. nach Tauchbehandlung mit einer 0,1 %igen waßrigen Zubereitung des Wirkstoffs auf nasses Fließpapier gelegt, wobei die Blattunterseite nach oben gerichtet ist. Nach Antrocknen der so behandelten Proben werden ungeflugelte Stadien von Aphis fabae aufgesetzt (etwa 100 pro Blattscheibe). Der Versuch wird dreimal wiederholt. Die Blattscheiben werden auf naß gehaltenem Filterpapier zwei Tage bei 25 °C und bei 16 Stunden Licht pro Tag aufgestellt. Dann wird die prozentuale Mortalität geschätzt und unter Bezug auf die unbehandelte Kontrolle wird nach Abbott die Wirkung berechnet.

Eine Wirkung von 80 % oder mehr hatten die Verbindungen gemäß den Beispielen Nr. 2, 4, 6, 7, 8 und 11 bis 22.

## Anwendungsbeispiel B

Wirkung der prophylaktischen Futterbehandlung gegen die Braune Reiszikade (Nilaparvata lugens Stål)

Reissämlinge (Oryza sativa L.) im Zweiblattstadium (etwa 10 je Polystyroltopf von 6,5 x 6,5 cm) werden unbehandelt bzw. nach Tauchbehandlung mit einer 0,1 % Wirkstoff enthaltenden wäßrigen Zubereitung bis zum Antrocknen der Flüssigkeit im Labor aufgestellt. Dann wird über jeden Topf ein Polystyrolzylinder gestülpt, durch dessen obere Öffnung etwa 30 mit Kohlendioxid betäubte Individuen von Nilaparvata lugens im 4.-5. Stadium eingebracht werden. Nach Verschließen der Öffnung mit einem engmaschigen Sieb werden die Töpfe zwei Tage lang bei 28 °C und bei 16 Stunden Licht pro Tag gehalten. Dann wird die prozentuale Mortalität bestimmt und unter Bezug auf die unbehandelte Kontrolle wird nach Abbott die Wirkung berechnet.

Eine Wirkung von 80 % oder mehr hatten die Verbindungen gemäß den Beispielen Nr. 1 bis 7, 11, 12, 18 und 23.

## Anwendungsbeispiel C

Wirkung der prophylaktischen Futterbehandlung gegen die Gemeine Bohnen-Spinnmilbe (Tetraychus urticae Koch)

Aus entwickelten Primarblättern der Buschbohne (Phaseolus vulgaris nanus Aschers.) werden runde Blattscheiben mit einem Durchmesser von 14 mm gestanzt und unbehandelt bzw. nach Tauchbehandlung mit einer 0,1 % Wirkstoff enthaltenden wäßrigen Zubereitung auf nasses Filterpapier gelegt, wobei die Blattunterseite nach oben gerichtet ist. Nach Antrocknen der so behandelten Proben werden sechs erwachsene Weibchen von Tetranychus urticae auf Jede Blattscheibe gesetzt und für 3 Tage bei 25 °C und bei 16 Stunden Licht pro Tag gehalten. Die Versuche werden viermal wiederholt. Dann werden die toten und lebenden Weibchen gezählt und entnommen. Gleichfalls werden die abgelegten Eier gezählt. Nach weiteren sieben Tagen werden die lebenden Larven gezählt und unter Bezug auf die unbehandelte Kontrolle wird nach Abbott die Gesamtwirkung berechnet.

Eine 80-100 %ige Wirkung hatten die Verbindungen gemäß den Beispielen Nr. 1 bis 9 und 11 bis 24.

## Anwendungsbeispiel D

Wirkung kurativer Blattbehandlung der Buschbohne (Phaseolus vulgaris nanus Aschers.) gegen Eier der Gemeinen Bohnenspinnmilbe (Tetraychus urticae Koch)

Im warmen Gewachshaus werden Samlinge der Buschbohne bis zur vollständigen Entwicklung der Primärblätter angezogen und dann mit adulten Weibchen von Tetranychus urticae besetzt. Einen Tag später werden die Pflanzen mit den inzwischen abgelegten Eiern mit einer 0,1 % Wirkstoff enthaltenden

wäßrigen Zubereitung tropfnaß gespritzt. Nach 7 Tagen bei etwa 25 °C wird der Anteil abgestorbener Eier an behandelten und unbehandelten Pflanzen bestimmt. Daraus wurde nach Abbott die Wirkung der Behandlung berechnet.

Eine 80-100 %ige Wirkung hatten die Verbindungen gemäß den Beispielen Nr. 1, 3, 8 und 11 bis 22.

## Anwendungsbeispiel E

Abtötende Wirkung auf Eier/Larven des Maiswurzelwurmes (Diabrotica undecimpunctata)

Die erfindungsgemäßen Verbindungen werden als wäßrige Zubereitung mit einer Wirkstoffkonzentration von 0,1 % eingesetzt. Von diesen Wirkstoffzubereitungen werden 0,2 ml auf den Boden einer Polystyrolpetrischale sowie auf den darin enthaltenen Maiskeimling und auf die im Schalenzentrum befindlichen ca. 50 Eier des Maiswurzelwurmes (Diabrotica undecimpunctata) pipettiert. Die verschlossenen Schalen werden bis zu 7 Tagen bei 25 °C unter Langtagbedingungen aufgestellt. Kriterium für die Wirkungsbeurteilung ist die Abtötung von Eiern oder der frisch schlüpfenden Larven bei Versuchsende.

Die Verbindungen gemäß den Herstellungsbeispielen 8, 16, 22 und 23 erzielten eine 80-100 %ige Wirkung.

## Anwendungsbeispiel F

Ovizide Wirkung auf Eiablagen der Baumwolleule (Heliothis virescens)

Die erfindungsgemäßen Verbindungen werden als wäßrige Zubereitung mit einer Wirkstoffkonzentration von 0,1 % eingesetzt. In diese Wirkstoffzubereitungen werden einen Tag alte Eiablagen, die von befruchteten Falterweibchen auf Filterpapier abgesetzt worden waren, bis zur völligen Benetzung getaucht und für vier Tage bei 25 °C unter Langtagbedingungen in geschlossenen Petrischalen deponiert. Kriterium für die Wirkungsbeurteilung ist die prozentuale Schlupfverhinderung im Vergleich zu unbehandelten Eiablagen.

Die Verbindungen gemäß den Herstellungsbeispielen 2, 5, 6, 7, 8, 11, 13, 16, 19, 22 und 23 erzielten eine 80 - 100 %ige Wirkung.

## Anwendungsbeispiel G

Insektizide Wirkung gegen die Schafsschmeißfliege (Lucilia sericata)

1 ml eines aliquoten Teiles einer Lösung der Testsubstanz in Aceton bei verschiedenen Konzentrationen wird auf Baumwoll-Dentalrollen (1 cm x 2 cm) aufgebracht, die sich in Glasröhrchen (2 cm 0 und 5 cm Länge) befinden. Nach Trocknung wird das so behandelte Material mit 1 ml einer Nährlösung getränkt, die von Junglarven der Schafsschmeißfliege (Lucilia sericata) durchsetzt ist. Dann werden die Glasröhrchen mit einem Baumwollstopfen verschlossen und für 24 Stunden bei 25 °C gehalten.

Die Auswertung der Versuche ergab, daß bei den Kontrollversuchen die Mortalität <5 % war. währenddessen die Verbindungen gemäß den Beispielen 11-14, 19 und 20 eine $LC_{50}$ bei 300 ppm oder weniger aufwiesen.

## Anwendungsbeispiel H

Akarizide Wirkung gegen Rinderzecken-Larven (Boophilus microplus)

Filterpapier (9 cm 0) wird mit 1 ml eines aliquoten Teiles einer Lösung der Testsubstanz in Aceton bei verschiedenen Konzentrationen getränkt. Nach Trocknung werden die Filterpapiere zu Umschlägen gefaltet, in welche Zecken-Larven (Boophilus microplus) eingebracht und für 48 Stunden bei 25 °C und 80 %

Raumfeuchte gehaltn werden. Anschließend wird die prozentuale Mortalität der Larven festgestellt und mit den Kontrollversuchen verglichen.

Die Kontrollversuche ergaben eine Mortalität von <5 %, währenddessen die Verbindungen gemäß den Beispielen 11 bis 15, 17, 18 und 21 eine 50 %ige Mortalität bei einer Konzentration von 300 ppm oder weniger ergaben.

## Anwendungsbeispiel I

Akarizide Wirkung gegen befruchtete weibliche Rinderzecken (Boophilus microplus)

Gruppen von je 5 ausgewachsenen weiblichen Zecken werden für 10 Minuten in Dispensionen wäßriger Acetonlösungen, die die Testsubstanzen und ein Netzmittel enthalten, getaucht. Anschließend werden die Zecken getrocknet, einzeln in Plastikbehälter gesetzt und bei 25 °C und 80 % Raumfeuchte gehalten. Anschließend wird die Mortalität der Zecken oder die Anzahl befruchteter und lebensfähiger Eier der überlebenden Zecken bestimmt. Die prozentuale Verringerung des vollständigen Fortpflanzungsvermögens (z.B. die Kombinationseffekte der Erwachsenenmortalität, verminderte Fruchtbarkeit und Mortalität der Eier) werden bestimmt und mit der Kontrolle verglichen.

Die Auswertung der Versuche ergab, daß bei den Kontrollversuchen die Verringerung des Fortpflanzungsvermögens <5 % war, währenddessen die Verbindungen gemäß den Beispielen 3, 11 und 15 eine mindestens 50 %ige Verringerung des Fortpflanzungsvermögens bei einer Konzentration von 500 mg/l oder weniger aufwiesen.

## Ansprüche

1. 2-Halogencyclopropylethanderivate der allgemeinen Formel I

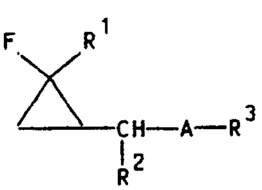

(I),

in der
$R^1$ Wasserstoff oder Chlor,
$R^2$ Wasserstoff, $C_1$-$C_6$-Alkyl, Aryl oder Aryl-$C_1$-$C_2$-alkyl,
A für die Gruppe $-CH_2-B-\underset{\underset{D}{\|}}{C}-$
oder $-\underset{\underset{O}{\|}}{C}-E$
steht.
(hierbei sind
$B-\overset{\overset{D}{\|}}{C}-$ oder E- mit $R^3$ verbunden, wobei B und D Sauerstoff oder Schwefel bedeuten),
E Sauerstoff, Schwefel oder den Rest $NR^4$ bedeuten und
$R^3$ und $R^4$ unabhängig voneinander für Wasserstoff, ein Alkalimetallatom oder ein entsprechendes Äquivalent eines zweiwertigen Metalls, $C_1$-$C_{20}$-Alkyl, $C_2$-$C_{20}$-Alkenyl $C_2$-$C_{20}$-Alkinyl, Halogen-$C_1$-$C_{10}$-alkyl, $C_3$-$C_6$-Cycloalkyl, $C_1$-$C_3$-Alkyl-$C_3$-$C_6$-cycloalkyl, $C_3$-$C_6$-Cycloalkyl-$C_1$-$C_6$-alkyl, Halogen-$C_3$-$C_6$-cycloalkyl-$C_1$-$C_6$-alkyl, Bicycloalkyl, Chlorfluorcyclopropylmethylcarbonyloxy-$C_1$-$C_{10}$-alkyl, Chlorfluorcyclopropylcarbonyloxy-$C_1$-$C_3$-alkoxy-$C_1$-$C_3$-alkyl, Aryl-$C_1$-$C_6$-alkyl, Aryl-$C_2$-$C_6$-alkenyl, Halogenaryl-$C_1$-$C_6$-alkyl, $C_1$-$C_4$-Alkylaryl-$C_1$-$C_4$-alkyl, Halogenaryl-$C_2$-$C_6$-alkenyl, Halogen-$C_1$-$C_4$-alkylaryl-$C_1$-$C_6$-alkyl, $C_1$-$C_3$-Alkoxyaryl-$C_1$-$C_6$-alkyl, Aryloxybenzyl, $\alpha$-$C_1$-$C_3$-Alkylphenoxybenzyl, Halogenphenyl(cyclopropyl)-$C_1$-$C_3$-alkyl, Halogenphenoxy-$C_1$-$C_6$-alkyl, Naphthyl-$C_1$-$C_6$-alkyl, freies Aryl oder durch $C_1$-$C_{20}$-Alkyl, Halogen-$C_1$-$C_6$-alkyl, $C_1$-$C_{16}$-Alkoxy, Halogen-$C_1$-$C_6$-alkoxy, Phenyl-$C_1$-$C_6$-alkyl, Phenyl-$C_1$-$C_6$-alkoxy, $C_3$-$C_{10}$-Cycloalkoxy, Halogen-$C_3$-$C_{10}$-cy-

cloalkoxy, $C_3$-$C_6$-Cycloalkylalkyloxy, Halogen-$C_3$-$C_6$-cycloalkyl alkyloxy, $C_2$-$C_6$-Alkenyloxy, Halogen-$C_2$-$C_6$-alkenyloxy, $C_2$-$C_6$-Alkinyloxy, Halogen-$C_2$-$C_6$-alkinyloxy, Alkylsulfonyloxy, Alkylphenylsulfonyloxy, Halogenalkylsulfonyloxy, Phenyl, Halogen, Amino, Cyano, Hydroxy, Nitro, Aryloxy, Heteroaryloxy, Halogenaryloxy, Arylamino, Halogenarylamino, $C_1$-$C_6$-Alkoxycarbonyl, $C_1$-$C_6$-Alkoxycarbonylmethyl, Halogen-$C_1$-$C_6$-alkoxycarbonyl,
$C_1$-$C_2$-Alkyldioxy, $C_1$-$C_6$-Alkylthio, Halogen-$C_3$-$C_6$-cycloalkylalkylamino, Halogen-$C_3$-$C_6$-cycloalkyl-alkylcarbonyloxy, $C_1$-$C_6$-Alkylamino, oder Di-$C_1$-$C_6$-alkylamino ein- oder mehrfach substituiertes Aryl, freies Heteroaryl, durch Halogen, $C_1$-$C_3$-Alkyl oder für Halogen-$C_1$-$C_3$-alkyl substituiertes Heteroaryl stehen (Alkyl steht für eine geradlinige oder verzweigte Kohlenstoffkette, Alkenyl steht für eine geradlinige oder verzweigte Kohlenstoffkette, die durch Doppelbindungen ein- oder mehrfach unterbrochen ist, Alkinyl steht für eine geradlinige oder verzweigte Kohlenstoffkette, die durch Dreifachbindungen ein- oder mehrfach unterbrochen ist, Aryl steht für einen ein- bis dreikernigen aromatischen Rest, Heteroaryl steht für einen 5- oder 6-gliedrigen Ring, der ein oder mehrere Stickstoff-, Sauerstoff- und/oder Schwefelatome enthält, gesättigt, teilgesättigt oder ungesättigt ist und gegebenenfalls benzoanelliert ist).

2. Zwischenprodukte zur Herstellung von 2-Halogencyclopropylethanderivaten der allgemeinen Formel I, dadurch gekennzeichnet, daß sie Verbindungen der allgemeinen Formel II

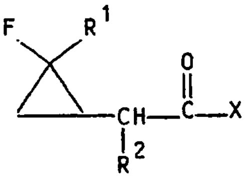

(II),

in der
$R^1$ und $R^2$ die in der Formel I angegebene Bedeutung haben und
X für Hydroxy, Chlor oder Brom steht, entsprechen.

3. Verfahren zur Herstellung von Verbindungen der allgemeinen Formel I, dadurch gekennzeichnet, daß man

a) falls A für die Gruppe

$-\overset{\overset{O}{\|}}{C}-E-$ steht, ein Säurehalogenid der allgemeinen Formel II

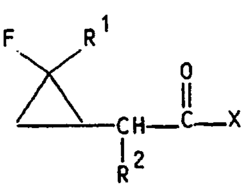

(II),

in der
$R^1$ und $R^2$ die in Formel I angegebene Bedeutung haben und
X für Chlor oder Brom steht,
mit einem Alkohol oder Amin der allgemeinen Formel III
$H - E - R^3$ (III),
in der
E und $R^3$ die in Formel I angegebene Bedeutung haben, gegebenenfalls in einem Lösungsmittel in Gegenwart eines Säureacceptors umsetzt, oder

b) eine freie Säure der allgemeinen Formel IV

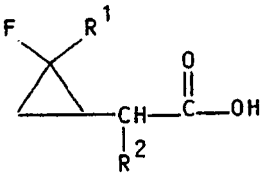

(IV),

in der $R^1$ und $R^2$ die in der Formel I angegebene Bedeutung haben, mit einem Alkohol oder Amin der Formel III, gegebenenfalls unter Verwendung eines Lösungsmittels in Gegenwart eines Katalysators, umsetzt, oder

c) eine Säure der allgemeinen Formel V

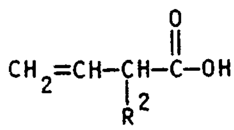

(V),

in der $R^2$ die in Formel I angegebene Bedeutung hat, mit einem Alkohol oder Amin der Formel III, gegebenenfalls in einem Lösungsmittel in Gegenwart eines Katalysators oder wasserentziehender Mittel, zu einer Zwischenverbindung der Formel VI

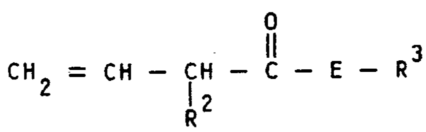

(VI),

in der E, $R^2$ und $R^3$ die in Formel I angegebene Bedeutung haben, umsetzt und diese in Gegenwart eines inerten Lösungsmittels mit Chlorfluorcarben reagieren läßt, oder

d) falls A die Gruppe $-CH_2-B-$ 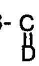 -

bedeutet, einen Alkohol der allgemeinen Formel VII

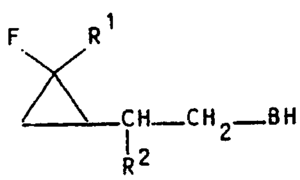

(VII),

in der $R^1$, $R^2$ und B die in Formel I angegebene Bedeutung haben, mit einer Säure der allgemeinen Formel VIII

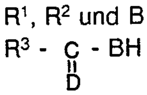 (VIII),

in der B, D und $R^3$ die in Formel I angegebene Bedeutung haben, gegebenenfalls unter Verwendung eines Lösungsmittels sowie eines Katalysators, umsetzt, oder

e) einen Alkohol der allgemeinen Formel VII mit einem Säurehalogenid der allgemeinen Formel IX

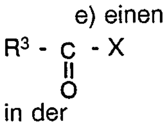 (IX),

in der X für Chlor oder Brom steht und

$R^3$ die in Formel I angegebene Bedeutung hat, gegebenenfalls unter Verwendung eines Lösungsmittels sowie eines Säureacceptors, umsetzt, oder

f) einen Alkohol der allgemeinen Formel X

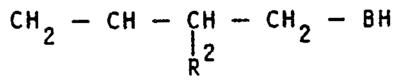 (X),

in der

B und $R^2$ die in Formel I angegebene Bedeutung haben, mit einer Säure der allgemeinen Formel VIII, gegebenenfalls unter Verwendung eines Lösungsmittels sowie eines Katalysators, zu einer Zwischenverbindung der Formel XI

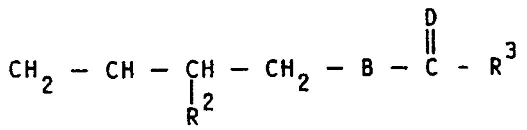 (XI),

in der

B, D, $R^2$ und $R^3$ die in Formel I angegebene Bedeutung haben, umsetzt und diese in Gegenwart eines inerten Lösungsmittels mit Chlorfluorcarben reagieren läßt, oder

g) einen Alkohol der allgemeinen Formel X mit einem Säurehalogenid der allgemeinen Formel IX, gegebenenfalls unter Verwendung eines Lösungsmittels sowie eines Säureacceptors, zu einer Zwischenverbindung der Formel XI umsetzt und diese in Gegenwart eines inerten Lösungsmittels mit Chlorfluorcarben reagieren läßt.

4. Schädlingsbekämpfungsmittel, gekennzeichnet durch einen Gehalt an mindestens einer Verbindung gemäß Anspruch 1 der allgemeinen Formel I.

5. Schädlingsbekämpfungsmittel gemäß Anspruch 4, in Mischung mit Träger- und/oder Hilfsstoffen.

6. Verwendung von Verbindungen gemäß Anspruch 1 der Formel I zur Bekämpfung von Insekten und Milben.